# EUROPEAN PATENT APPLICATION

(11) **EP 1 721 621 A1**
(43) Date of publication of application: **15.11.2006**
(21) Application number: 05010047.8
(22) Date of filing: 09.05.2005
(51) Int. Cl.: A61L 9/12, A61L 9/04

(54) **Perfume releasing packages**

(71) Applicant: THE PROCTER & GAMBLE COMPANY, Cincinnati, Ohio 45202 (US)
(72) Inventor: Burrowes, Lee, Horsell Woking - Surrey GU21 4XH (GB); Evangelista, Olindo, 66023 Francavilla al Mare (Chieti) (IT)
(74) Representative: Veronese, Pancrazio

(57) **Abstract**

Article of manufactures comprising a packaged sealed product (10) and an improved system to release a perfume without unsealing the product. The articles comprise an enclosure (40) formed outside the packaged product (10) which contains a perfume releasing material (45). The enclosure (40) is saturated with a perfumed headspace and comprises means to release the perfumed headspace to the external environment and means to regenerate the headspace for the next use.

## Description

### Field of the Invention

The present invention relates to innovative packaged products wherein the package is able to release fragranced air in response to an action from a user e.g. when a consumer picks up said packaged product in a store, without the need to open the package and expose the product to the air.

### Background of the Invention

Modem studies on consumer behaviour show that consumers give more and more importance, when selecting which product to buy, to a pleasant shopping experience which involves all senses. Stores, shelves, product packages and labels are all designed in order to provide the consumer not only with information on the product contained in the package, but also to provide a pleasant experience to the consumer touching and handling the product before purchase.

Smell is one of the key elements providing this experience, for example in many cases consumers want to test the smell of a product before purchasing it so they tend to open packages and bottles to perform this trial. On the other hand product manufacturers tend to seal the products to avoid product contamination, dispersion or alteration, thus inhibiting consumers to make this test. It is therefore important to be able to provide consumers with a way to test the smell of a sealed product before actually purchasing it, without having to break the seal or open the package.

In other cases product manufacturers may want to expose consumers to a smell which is not the smell of the product (e.g. if the product has no smell), but is instead a perfume or a fragrance that the product manufacturers consider part of the experience the consumer has to make when handling that product.

Conventional systems to integrate a perfume releasing material in a package commonly involve the use of perfumed stickers or labels typically applied to the external surface of the package where the perfume is encapsulated and the consumer is directed to peel off or to scratch the label.

These systems however suffer severe limitations e.g. peel labels cannot be reused once peeled off, so that the item with the label peeled off is perceived by the consumer as a damaged item and is often refused by the consumer. Similarly, scratch and sniff labels tend to wear out and to lose effectiveness after some uses thus losing functionality and appeal for the consumer.

JP06001332 by Sanra KK, attempts to solve this problem by suggesting a container wherein at least a part of the external surface in contact with the air is provided with a polymeric material comprising a perfume. This solution has still many disadvantages in fact the container when exposed in a store will continuously release the perfume in the environment mixing it with the perfume released by other products employing the same technology. Additionally this continuous and unnecessary release of perfume will lead to perfume depletion in short time. Moreover retailers and consumers would refuse to have in the shop or at home several containers each continuously releasing their own perfume and mixing up in the air.

There is therefore a need for new systems to provide a scent to consumers handling a packaged product which are activated by the consumer.

### Summary of the Invention

In a first embodiment the present invention encompasses an article of manufacture comprising:
a) a product
b) a package separating said product from the external environment.
c) an enclosure formed outside said package and between said package and a second surface, said enclosure comprising a headspace and a perfume releasing material which releases a perfume in said headspace,
d) said article comprising means for releasing said perfume in said headspace to the external environment and means to regenerate said headspace collecting air from the external environment.

In a second embodiment the present invention encompasses an article of manufacture comprising
a) a product
b) a package separating said product from the external environment
c) said package comprising a base
d) said base comprising a concave portion
e) said concave portion comprising a perfume releasing material.
f) said concave portion being able to cooperate with a second surface to form an enclosure.

### Brief Description of the Drawings

Figure 1 represents an enlarged side view of the top portion of an article according to the present invention, a deodorant can, wherein the cap is shown in a section taken along a plane comprising the central longitudinal axis of the can.
Figure 2 represents an enlarged perspective view of the top portion of an article, a shampoo bottle, according to the present invention wherein the plastic lid is shown in a section taken along a plane comprising the central longitudinal axis of the bottle.
Figure 3 represents an article according to the present invention, an air freshener in a thermoformed blister pack, in a perspective view.
Figure 4 represents an article according to the present invention, a toothpaste tube in a carton box, in a perspective view wherein the carton box is shown in transparency.
Figure 5 represents an article according to the present invention, a deodorant can placed on a flat surface wherein base portion is illustrated as a sectional view taken along a plane comprising the central longitudinal axis of the can.

### Detailed Description of the Invention

The present invention encompasses an article of manufacture comprising a product in a package. For package it is intended any physical barrier separating said product from the external environment including for example a bottle, a can, a film wrap, a case, a tube (e.g. for toothpaste, glue, sauce), a box, and the like. Said package is preferably a sealed package i.e. a package which completely isolates said product from the external environment and which is intended to remain sealed until the product is purchased by a consumer. For product it is intended any article which can be commercialized to final consumers, e.g. in a supermarket, such as, for example, but not limiting to, household detergent, personal detergent, personal care products, cosmetics, hygiene articles, food products, household goods, furniture, outdoor goods, apparel goods and the like.

In a first embodiment said article of manufacture further comprises an enclosure formed outside of said package and between said package and a second surface. For enclosure it is meant a delimited space whose boundaries are defined by the external surface of said package and a second surface as defined below. Said enclosure is in general "closed" in the sense that there is no substantial exchange of air from inside the enclosure to the outside in absence of a specific action from the user. In some embodiments of the present invention the enclosure is tightly closed (e.g. a cap over the seal of a sealed bottle) in other embodiments said enclosure will comprise at least one small opening through which the air contained in the enclosure can be expelled only in response to an action from a user such as pushing or squeezing at least a portion of the article.

Said second surface can be any surface which delimits the enclosure together with a portion of the external surface of said package.

Example of second surfaces are e.g. the internal surface of a cap or a lid over the seal of a sealed bottle, of a secondary package which partially or entirely encases said package (a carton box, a thermoformed blister pack and the like), or a re-attachable label closing a concave portion of said package surface.

Said enclosure comprises a headspace. The headspace is defmed herein as the volume of air comprised in said enclosure. Said enclosure also comprises a perfume releasing material. The perfume is released in the enclosure creating an equilibrium state wherein the air in the enclosure headspace is saturated with perfume. Once equilibrium is reached, due to the low or minimal exchange of air from within the enclosure to the external environment, the perfume releasing material stops releasing the perfume.

The articles of the present invention also comprise means for releasing said perfume in said headspace to the external environment and to regenerate said headspace collecting air from the external environment. Said means are preferably activatable by a consumer handling the article in a store typically before purchase.

Any means capable to achieve the intended result of releasing the perfumed air from the headspace to the external environment and then regenerating the headspace is suitable in the articles of the present invention. Some non limiting examples of these means include a lid or a cap over a the seal of a sealed bottle or container, a thermoformed blister pack completely encasing the packaged product comprising at least one small hole or aperture, a carton box in which the packaged product is closed (e.g. a carton box enclosing a toothpaste tube), a flexible package surrounding the packaged product comprising at least a small hole or aperture and the like.

For the purpose of clarity, preferred configurations within said first embodiment can be collected in two main groups:

In a first group the packaged product is in a sealed package, said sealed package comprising a cap or a lid, which do not contribute to sealing said product, said cap or lid defining the enclosure comprising the headspace to be saturated with the perfume. The perfume releasing material is applied in the so defined enclosure, for example onto the external surface of the package or, more preferably, on the internal surface of the cap or lid. A consumer in a store can remove the cap or lift the lid without unsealing the packaged product thus causing the release of the perfumed air in the headspace. When the cap or the lid is closed again, a fresh headspace is regenerated. The perfume releasing material will start releasing perfume in the headspace until equilibrium is reached so that the headspace is regenerated, ready to release a new perfume bloom upon a subsequent opening of the cap or lid.

A second group of preferred configurations are those activatable by pushing or squeezing the article of manufacture of the present invention. This group includes all cases wherein a secondary package completely encases the packaged product. Said headspace is formed between the external surface of the packaged product and the internal surface of the secondary package, which corresponds to said second surface, and the perfume releasing material is preferably applied onto at least one of said packaged product external surface or secondary package internal surface. Any material commonly used for packaging purposes can be used to manufacture said secondary package, for example, but non limited to carton, plastic, wood, plastic films, metal films, composite materials, carton/plastic, or carton/metal film laminates and the like. Said secondary package is not sealed and preferably flexible. Means for releasing the perfume from the headspace to the external environment, and also for regenerating the headspace collecting air from the external environment may comprise a re-closable opening in said secondary package, wherein the user can open the secondary package and be exposed to the perfume, but without exposing the product to the external environment, and then re-close it thus regenerating the headspace. More preferably said secondary package comprises at least one small hole or aperture such that there is no substantial exchange of air from inside the enclosure to the external environment. Preferably the surface area, measured along the surface of said secondary package, of said at least one hole or aperture is less than 30 mm², more preferably less than 10 mm² even more preferably less than 5 mm², most preferably less than 2mm². A consumer squeezing the article will cause a part of the perfumed air in the headspace to be released, while the elasticity of the container will return it to its original shape upon release of said pressure by the consumer thus regenerating said headspace.

A less preferred configuration in this family includes a blister or a small vented button applied onto the surface of the packaged article e.g. on the side surface or on the top of the cap. Perfume releasing material is contained within said blister. The small button or blister can be made of compressible plastic and has at least a hole or aperture to release the perfumed air in the headspace. Preferably the surface area, measured along the surface of said button or blister, of said at least one hole or aperture is less than 30 mm², more preferably less than 10 mm² even more preferably less than 5 mm², most preferably less than 2mm². This embodiment is less preferred because said second surface requires the use of a further material only devoted to create said second surface. It is in general preferred that said second surface has another function other than that of defining said enclosure such as e.g. being the internal surface of a cap or of a secondary package.

In a second embodiment the articles of manufacture of the present invention comprise a packaged product wherein said package further comprises a package base. "Package base" is intended as a portion of the surface on which said article is able to stand when placed on a substantially-flat surface.

Said package base further comprises a concave portion. Said concave portion is able to cooperate with a second surface to form an enclosure. Preferably said package base comprises a series of coplanar points forming a closed perimeter in which said concave portion is comprised so that when the article of the present invention is placed onto a flat surface said coplanar points cooperate with said flat surface forming an enclosure. A preferred example of this embodiment is a typical aerosol can which has a concave base with a circular profile. Said circular profile is commonly made of coplanar points so that, when the can is placed onto a flat surface, said circular profile forms the enclosure between said concave base and said flat surface. Although not preferred, other configurations can be envisioned by those skilled in the art, for example the package could have a not planar profile which cooperates forming a joint with a correspondingly formed slot onto a surface thus forming the enclosure. An example of this embodiment is an aerosol can having a wavy base instead of the usual circular base which can be placed on a rack having corresponding slots.

Said concave portion comprises a perfume releasing material, preferably applied onto at least a portion of the surface of said concave portion.

When an article of the present invention is placed onto said surface forming an enclosure, said enclosure comprises a headspace (i.e. a volume of air comprised in said enclosure), perfume is released in the enclosure creating an equilibrium state wherein air in the enclosure headspace is saturated with perfume. Once equilibrium is reached, due to the low or minimal exchange of air from within the enclosure to the external environment, the perfume releasing material stops releasing the perfume.

When a consumer lifts said article from the surface on which it is placed, the perfumed air comprised in the enclosure is released to the external environment and the consumer perceives the perfume. When the consumer places the article back on said second surface the headspace is regenerated for next perfume bloom.

In all embodiments, artwork on the article may be used to communicate the scent to the consumer, e.g. a picture of a lemon with lemon scent being delivered from a separate perfume release material.

According to the present invention, the perfumed headspace within the enclosure is always kept isolated from the product, hence avoiding any contamination between the perfume releasing material and the product. The present invention has the further advantage that, at the same time, the perfume releasing material is made available to a consumer, i.e. released in the environment, only upon direct activation from the consumer, while being normally isolated from the environment.

Perfume releasing materials for use in the present invention can be provided in any suitable form. In some embodiments, the scent releasing material comprises perfumes, such as perfume oils, that are incorporated onto or into a suitable carrier. Any perfume can be used in the present invention. The carriers can be provided in the following non-limiting forms: a solid, a gel, beads, encapsulates, wicks, a carrier material, such as a porous material impregnated with or containing the scent, and combinations thereof. A foam or sponge material impregnated or containing the perfume can be used as a carrier and is particularly suitable for those embodiments of the present invention which are activated by pushing or squeezing the article of manufacture as described above. In some embodiments the carrier is in the form of a gel which together with the perfume forms a gel composition. Two non-limiting examples of gels that can be used are hydroxypropyl cellulose and fumed silica. The perfumes are often formulated into a gel composition to reduce the partitioning effect (i.e. the effect which causes that the more volatile components of a perfume tend to evaporate more than the less volatile components thus leading to a change in perfume character with time) that occurs when a perfume evaporates. The amounts of perfume and gel in the gel composition can vary depending on the particular perfume and the gel. In certain non limiting embodiments, the gel composition is about 90% perfume and about 10% hydroxypropyl cellulose or about 93% perfume and about 7% fumed silica, although other ratios are clearly contemplated. There are a variety of hydroxypropyl cellulose/silica ratios that may be used in combination in the gel composition as well as other appropriate gelling agents.

Particularly preferred perfume carriers for use in the present invention comprise polymeric materials in which perfumes are solubilized. In general these materials are able to absorb and gradually release large amounts of perfumes for very long times. In addition the partitioning effect is further reduced with respect to gels.

Any polymeric material capable to solubilize and gradually release a perfume can be used as a perfume carrier in the present invention. Polymeric materials which can be used for this purpose are those comprising e.g. PVC polymers, polyurethane polymers and co-polymers, polysaccharide polymers and co-polymer, polyoxyalkylene polymers and co-polymers, co-polymers formed by a monomer comprising an ether group and a monomer non comprising an ether group, copolymer of ethylene with at least another monomer comprising at least a heteroatom. Particularly preferred polymeric materials are those comprising polyether-amide co-polymers, polyether-ester copolymers, polyurethanes, ethylene vinyl acetate co-polymers, polytetramethylene glycol. Even more preferred are those comprising ethylene vinyl acetate co-polymers.

Particularly preferred perfume carriers are polymeric compositions which, in addition to one or more polymers as described above also comprise one or more plasticizers. Plasticizers reduce the melting point of the material, thus allowing to incorporate perfumes at lower temperatures thus minimizing perfume loss e.g. when the perfume releasing material is applied in the molten state to the desired substrate. Plasticizers also reduce the viscosity of the melt, thus allowing to handle the perfume releasing material as a hot melt glue typically in standard hot melt coating apparatuses in a manufacturing line thus significantly simplifying the manufacturing process of any article comprising such perfume releasing materials. Moreover the skilled man, by choosing the appropriate plasticizer or mixture of plasticizers among those which are suitable for a certain polymeric matrix, can modify the polarity of the polymeric matrix in order to match the polarity of the perfume thus allowing to incorporate a larger amount of perfume and a broader variety of perfume ingredients.

Suitable plasticizers for use in the polymeric compositions according to the present invention include citric acid esters, low molecular weight polyesters, polyethers, liquid rosin esters, aromatic sulphonamides, phthalates, benzoates, sucrose esters, derivatives of polyfunctional alcohols (where "polyfunctional" means having 2 or more hydroxyl groups),-adipates, tartrates, sebacates, esters of phosphoric acid, fatty acids and diacids, fatty alcohols and diols, epoxidised vegetable oils etc, and mixtures thereof. The different polarity of the different compatible plasticizers (measurable with any method known to those skilled in the art, for example water/octanol partition coefficient) can be used to tune the polarity of the polymeric matrix in order to provide a better match with the polarity of the perfume.

Preferred polymeric compositions comprising polymers, plasticizers and perfumes as mentioned above are described in co-pending European Patent Applications number 03026234.9 filed on November 14, 2003, 04013513.9 filed on June 8, 2004, 04018573.8 filed on August 5, 2004 and 04026243.8 filed on November 11, 2004 all assigned to The Procter & Gamble Company.

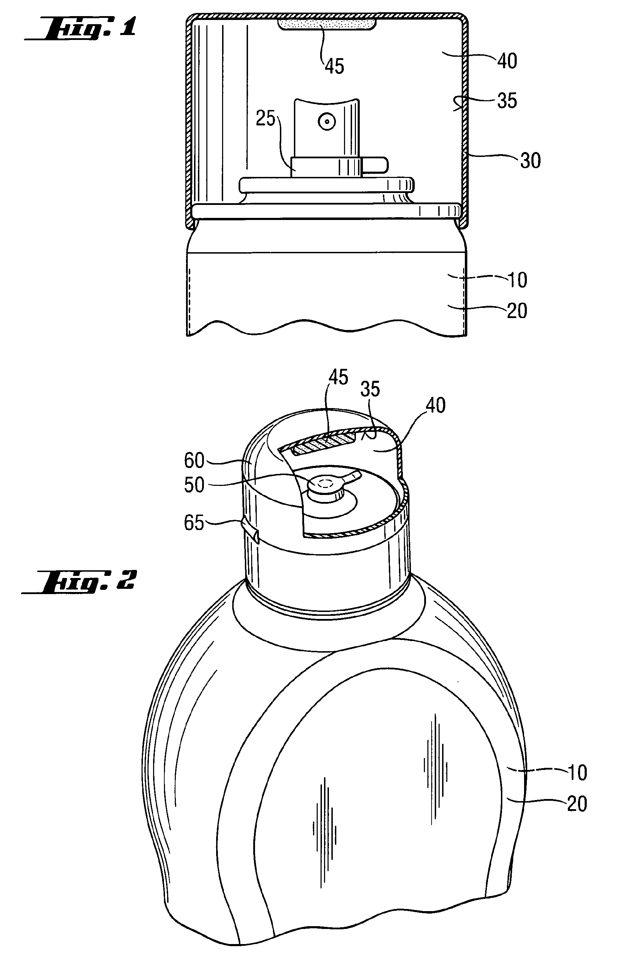

Some examples of articles according to the present invention are described below in examples 2 to 6 and shown in Figures 1 to 5.

All articles in the examples 2 to 6 comprise a perfume releasing material according to the following:

### Example 1 - Perfume releasing material

24.75 parts of Elvax® 250, a poly(ethylene-co-vinyl acetate) with a vinyl acetate content of 28 wt% and a melt flow index of 25 dg/min (ASTM D1238), available from Dupont, 9.75 parts of Escorene™ Ultra MV 02528, a poly(ethylene-co-vinyl acetate) with a vinyl acetate content of 27.5 wt% and a melt viscosity at 190 °C of 3100 cps (ExxonMobil method), available from ExxonMobil Chemical, 15 parts of ForalynTM 5020F, a rosin ester plasticiser available from Eastman Chemical and 0.5 parts of IrganoxTM B225, an antioxidant available from Ciba Geigy (Switzerland) were added to a sigma blade mixer and heated to a temperature of about 10-20 °C above the melting point of the polymer (about 120 °C). The ingredients were mixed until a homogeneous mass was obtained. The temperature was then reduced to a point where the mixture was still molten, typically to about 10-20 °C above the melting point of the mixture (about 80 °C in the present case). 50 parts of benzyl acetate, a perfume material available from Sigma Aldrich, were added to the plasticised polymer mixture. The ingredients were mixed until a homogeneous mixture was obtained, and the resultant material removed from the mixer, cooled to room temperature is used as a perfume releasing material to be applied as a hot melt adhesive.

### Example 2

A product 10, a deodorant composition, is contained in a package 20, a pressurized aerosol can, comprising a spray nozzle which is sealed with a plastic seal (25) wherein a consumer should break the seal in order to spray some product. Said aerosol can has a plastic cap 30. An enclosure 40 is formed between said package 20 (the can) and a second surface 35 which is the internal surface of said cap 30. 2 grams of perfume releasing hot melt adhesive 45 according to Example 1 are attached to the internal surface 35 of said cap 30. The enclosure 40 is saturated with a perfumed headspace. The cap 30 can be removed by a consumer in a shop, releasing said perfumed headspace to the external environment. When the cap is closed a new headspace is formed into said enclosure 40 which is quickly saturated with perfume regenerating said perfumed headspace for next use. Figure 1 represents an enlarged side view of the top portion of an article according to Example 2 wherein the cap 30 is shown in a section taken along a plane comprising the central longitudinal axis of the can.

### Example 3

A product 10, a shampoo, is contained in a package 20, a plastic bottle, comprising an opening which is sealed with an aluminum foil seal 50 and a plastic lid 60 joined to the main body of the bottle via a hinge 65. An enclosure 40 is formed between said package 20 (the bottle) and a second surface 35 which is the internal surface of said plastic lid 60. 2 grams of perfume releasing hot melt adhesive 45 according to Example 1 are attached to the internal surface 35 of said plastic lid 60. The enclosure 40 is saturated with a perfumed headspace. The lid 60 can be opened by a consumer in a shop, releasing said perfumed headspace to the external environment. When the lid is closed a new headspace is formed into said enclosure 40 which is quickly saturated with perfume regenerating said perfumed headspace for next use. Figure 2 represents an enlarged perspective view of the top portion of an article according to Example 3 wherein the plastic lid 60 is shown in a section taken along a plane comprising the central longitudinal axis of the bottle.

### Example 4

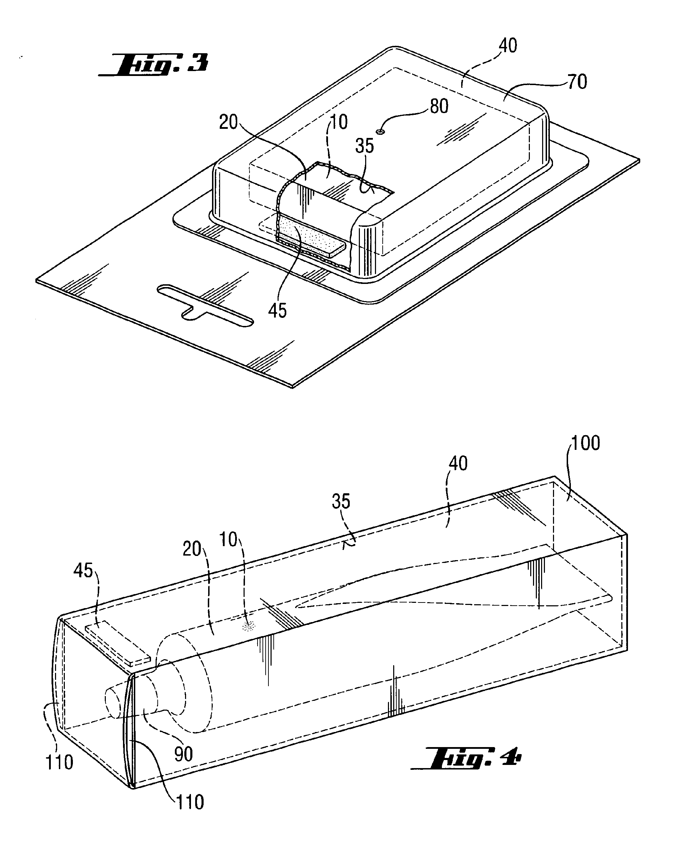

A product 10, an air freshener, is contained in a package 20, a polyethylene wrap, which completely seals said product. Said package is contained in a secondary package, a thermoformed blister pack 70, which completely encases said package 20. An enclosure 40 is formed between said package 20, the polyethylene wrap, and a second surface 35, which is the internal surface of said thermoformed blister pack 70. 2 grams of perfume releasing hot melt adhesive 45 according to Example 1 are attached to the internal surface 35 of sail blister pack 70. The enclosure 40 is saturated with a perfumed headspace. The blister pack 70 comprises a small circular hole 80 having a diameter of about 2 mm. A consumer handling the article can release said perfumed headspace to the external environment via said hole 80 by pressing said blister pack 70. When pressure is released said blister pack takes back its original shape such that said enclosure 40 is quickly saturated with perfume regenerating said perfumed headspace for next use. Figure 3 represents an article according to Example 4 in a perspective view.

### Example 5

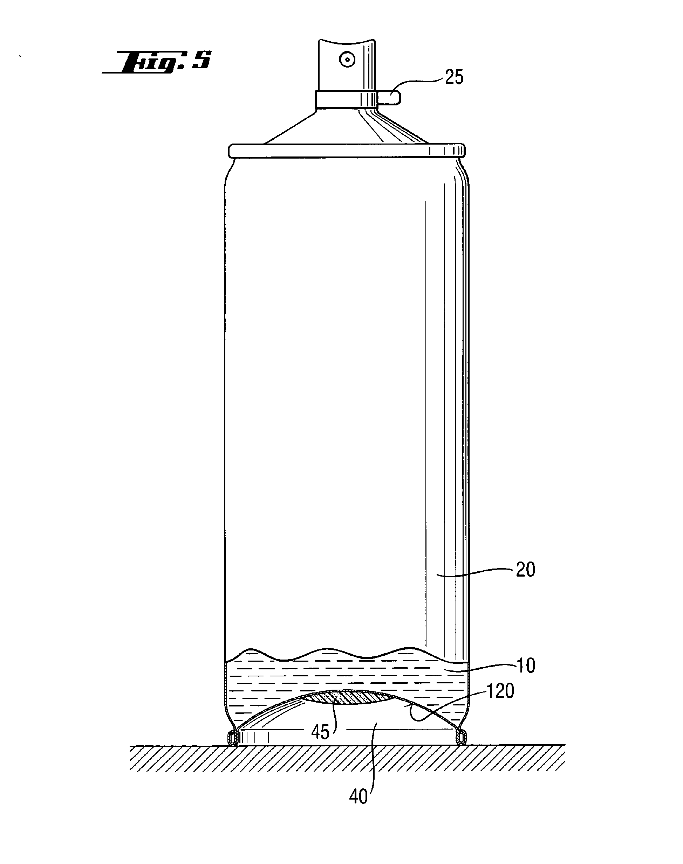

A product 10, a toothpaste, is contained in a package 20, the toothpaste tube. Said tube is sealed with a screwed plastic cap 90 which completely seals said product 10. Said package is contained in a secondary package 100, a carton box, which completely encases said package 20. An enclosure 40 is formed between said package 20, the toothpaste tube, and a second surface 35 which is the internal surface of said carton box 100. 2 grams of perfume releasing hot melt adhesive 45 according to Example 1 are attached to the internal surface 35 of said carton box 100. The enclosure 40 is saturated with a perfumed headspace. The carton box 100, being made by folded cardboard, forms two openings 110 on the openable side. A consumer handling the article can release said perfumed headspace to the external environment via said openings 110 by pressing said carton box 100. When pressure is released said carton box takes back its shape such that said enclosure 40 is quickly saturated with perfume regenerating said perfumed headspace for next use. Figure 4 represents a perspective view of article according to Example 5 wherein the carton box 100 is shown in transparency.

### Example 6

A product 10, a deodorant, is' contained in a package 20, a pressurized aerosol can, comprising a spray nozzle which is sealed with a plastic seal (25) wherein consumer should break the seal in order to spray some product. Said aerosol can has a concave base 120. 2 grams of perfume releasing hot melt adhesive 45 according to Example I are attached onto the surface of said base 120. When said can 20 is placed onto a shelf in a shop an enclosure 40 is formed between said concave base 120 and the shelf. The enclosure 40 is saturated with a perfumed headspace. When a consumer in the shop lifts the can from the shelf said perfumed headspace is released to the external environment. When said can is placed back on the shelf the enclosure 40 is again provided and a new headspace is formed into said enclosure 40 which is quickly saturated with perfume regenerating said perfumed headspace for next use. Figure 5 represents an article according to Example 6 when placed on a flat surface such as a shelf in a shop wherein base portion is illustrated as a sectional view taken along a plane comprising the central longitudinal axis of the can in order to show the concave base 120.

## Claims

1. An article of manufacture comprising:
a) a product
b) a package separating said product from the external environment.
c) an enclosure formed outside said package and between said package and a second surface, said enclosure comprising a headspace and a perfume releasing material which releases a perfume in said headspace,
d) said article comprising means for releasing said perfume in said headspace to the external environment and means to regenerate said headspace collecting air from the external environment.

2. An article of manufacture comprising
a) a product
b) a package separating said product from the external environment
c) said package comprising a base
d) said base comprising a concave portion
e) said concave portion comprising a perfume releasing material.
f) said concave portion being able to cooperate with a second surface to form an enclosure.

3. An article according to claim 1, wherein said second surface is the internal surface of a cap or of a lid over a sealed container.

4. An article according to claim 1 wherein said second surface is the internal surface of a secondary package completely encasing said package.

5. An article according to claim 4 wherein said secondary package is a box.

6. An article according to claim 4 wherein said secondary package is a thermoformed blister pack.

7. An article according to claims .4, 5 or 6 wherein said secondary package comprises at least one small hole or aperture.

8. An article according to claim 2, wherein said package base comprises a series of coplanar points forming a closed perimeter.

9. An article according to any of claims 1 to 8 wherein the perfume releasing material comprises a perfume and at least one polymer.

10. An article according to claim 9 wherein the perfume releasing material comprises at least one plasticizer.
